# EUROPEAN PATENT SPECIFICATION

(11) **EP 4 506 011 B1**
(45) Date of publication and mention of the grant of the patent: **03.12.2025**
(21) Application number: 23783290.2
(22) Date of filing: 31.03.2023
(51) Int. Cl.: A61K 35/32, A61P 21/00, A61P 25/28, C12N 5/0797

(54) **COMPOSITIONS FOR TREATING INTRACTABLE NEUROLOGICAL DISEASES AND METHODS FOR THEIR PRODUCTION**
FEUERFESTE ZUSAMMENSETZUNG ZUR BEHANDLUNG VON NEUROLOGISCHEN ERKRANKUNGEN UND VERFAHREN ZUR HERSTELLUNG DAVON
COMPOSITION DE TRAITEMENT DE TROUBLE NEUROLOGIQUE RÉFRACTAIRE ET SON PROCÉDÉ DE PRODUCTION

(30) Priority: 07.04.2022 JP 2022064178
(43) Date of publication of application: 12.02.2025
(73) Proprietor: Saiseiken Co., Ltd., Zushi-shi, Kanagawa 249-0008 (JP)
(72) Inventor: UEDA, Minoru, Zushi-shi Kanagawa 249-0008 (JP)
(74) Representative: Hasegawa, Kan
(86) International application number: PCT/JP2023/013671
(87) International publication number: WO 2023/195432

(56) References cited:
- WO-A1-2011/118795
- WO-A1-2020/130038
- CN-B- 107 828 722
- JP-A- 2005 334 456
- JP-A- 2019 508 068
- JP-A- 2022 502 032
- JP-A- 2022 502 033
- KR-A- 20090 008 784
- UEDA TOMOYUKI ET AL: "Stem Cells From Human Exfoliated Deciduous Teeth-Conditioned Medium (SHED-CM) is a Promising Treatment for Amyotrophic Lateral Sclerosis", FRONTIERS IN PHARMACOLOGY, vol. 13, 3 February 2022 (2022-02-03), CH, XP093249429, ISSN: 1663-9812, DOI: 10.3389/fphar.2022.805379
- YONG SEUNG LEE ET AL: "Exosome-Mediated Ultra-Effective Direct Conversion of Human Fibroblasts into Neural Progenitor-like Cells", ACS NANO, vol. 12, no. 3, 20 February 2018 (2018-02-20), US, pages 2531 - 2538, XP055604604, ISSN: 1936-0851, DOI: 10.1021/acsnano.7b08297
- UEDA, MINORU: "Organ regeneration using stem cell culture medium", JOURNAL OF THE SOCIETY FOR JAPANESE BLOOD PROGRAMME, JAPAN SOCIETY FOR HEMATOLOGY, JP, vol. 36, no. 4, 1 January 2014 (2014-01-01), JP , pages 841 - 844, XP009550178, ISSN: 0917-7833
- MITA, TSUNEYUKI ET AL.: "F-1-4: Possibility of treating Alzheimer's dementia using deciduous dental pulp stem cell-derived culture supernatant", REGENERATIVE MEDICINE, JAPANESE SOCIETY FOR REGENERATIVE MEDICINE, JP, vol. 12, no. Suppl., 1 January 2013 (2013-01-01), JP , pages 266, XP009550180, ISSN: 1347-7919
- GAO QIANHUA, WALMSLEY A. DAMIEN, COOPER PAUL R., SCHEVEN BEN A.: "Ultrasound Stimulation of Different Dental Stem Cell Populations: Role of Mitogen-activated Protein Kinase Signaling", JOURNAL OF ENDODONTICS, ELSEVIER, AMSTERDAM, NL, vol. 42, no. 3, 1 March 2016 (2016-03-01), AMSTERDAM, NL , pages 425 - 431, XP093097196, ISSN: 0099-2399, DOI: 10.1016/j.joen.2015.12.019
- DRAGO DENISE; COSSETTI CHIARA; IRACI NUNZIO; GAUDE EDOARDO; MUSCO GIOVANNA; BACHI ANGELA; PLUCHINO STEFANO: "The stem cell secretome and its role in brain repair", BIOCHIMIE, MASSON, PARIS, FR, vol. 95, no. 12, 1 July 2013 (2013-07-01), FR , pages 2271 - 2285, XP028768030, ISSN: 0300-9084, DOI: 10.1016/j.biochi.2013.06.020
- CHIAKI SHIMOJIMA, ET AL.: "Conditioned Medium from the Stem Cells of Human Exfoliated Deciduous Teeth Ameliorates Experimental Autoimmune Encephalomyelitis", THE JOURNAL OF IMMUNOLOGY, WILLIAMS & WILKINS CO., US, vol. 196, no. 10, 15 May 2016 (2016-05-15), US , pages 4164 - 4171, XP055603827, ISSN: 0022-1767, DOI: 10.4049/jimmunol.1501457

## Description

### Technical Field

This invention relates to compositions for treating intractable neurological diseases and methods for their production.

### Background of the invention

Intractable neurological diseases include amyotrophic lateral sclerosis (ALS) and Alzheimer's disease. Amyotrophic lateral sclerosis (ALS) is a neurodegenerative disease that selectively and systematically affects upper and lower motor neurons. Muscle atrophy begins in one upper extremity and progresses to the contralateral upper extremity and both lower extremities, during which time the whole body tenses up due to speech impairment, dyspnea, and spasticity of the extremities. Without ventilator management, death occurs within 2 to 5 years of onset due to respiratory failure.

The cause of ALS is unknown and there is no effective treatment. Therefore, there have been no reports of successful treatments in the past. Once the disease develops, there is no way to alleviate the symptoms or stop the progression of the disease, making ALS the cruelest of all neurological diseases.

The number of Alzheimer's disease patients worldwide will reach 76 million by 2030 and 135 million by 2050. In Alzheimer's disease, a protein called amyloid beta protein accumulates in the brain, and a further protein called tau accumulates, resulting in a decrease in nerve cells and brain atrophy. The hippocampus in the brain is known as the center of memory. In Alzheimer's disease, brain atrophy begins near the hippocampus and spreads. Therefore, symptoms begin with memory impairment and gradually decline in overall cognitive function. There is no effective treatment for Alzheimer's disease, and it has become a major social problem.

Regenerative medicine using stem cells is attracting attention as a versatile alternative technology for diseases that are difficult to treat with conventional medical technology. Stem cell-based regenerative medicine is a promising tool in a new clinical platform for all intractable diseases. There are many diseases for which regenerative medicine can be applied or is expected to be applied, and numerous studies are underway for clinical application. Among them, intractable neurodegenerative diseases such as amyotrophic lateral sclerosis (ALS) are one of the most promising diseases to be treated by regenerative medicine.

Various types of stem cells have been reported, including somatic stem cells, human fetal stem cells, embryonic stem cells (ES cells), and induced pluripotent stem cells (iPS cells). There are somatic stem cells derived from bone marrow or fat (Non-Patent Document 1), but these stem cells have several disadvantages: (1) the number of harvestable stem cells decreases with age, (2) it is difficult to ensure the safety of transplanted stem cells due to the accumulation of genetic mutations during aging, (3) cell proliferation ability is low, and (4) the collection of stem cells involves severe invasion of the body. To solve these problems, it is important to develop new stem cell resources for the treatment of intractable neurological diseases. Transplantation therapy of intractable neurological diseases using neural stem cells derived from human fetuses or ES cells (Patent Document 1) is recognized as a realistic research agenda, but it faces major ethical and safety issues. In addition, concerns about the carcinogenicity of ips cells have not been dispelled, and none of these are practical stem cell sources.

Under these circumstances, the inventors have found that compositions containing stem cell culture conditioned medium, especially those obtained by serum-free culture of dental pulp stem cells, but not containing dental pulp stem cells, are effective in healing skin damage, restoring motor function and reducing infarct volume in mice with cerebral infarction, inducing neurite outgrowth of nervous system cells, and in inhibiting apoptosis. apoptosis inhibition, and spinal cord injury healing effects (Patent Document 2).
The conditioned medium does not contain the cells themselves, and thus does not cause immune rejection. Therefore, culture supernatants made from other people's stem cells can be used without concern for rejection, and the range of application is extremely high. In addition, if the culture supernatant is stored in advance, it can be used immediately when needed, which is highly convenient.

It has been reported that ultrasound irradiation of cultured chondrocytes enhances the biosynthesis of cartilage matrix (Non-Patent Document 2). Ultrasound, which produces such effects, has been investigated by Duarte et al. for the treatment of bone fractures (Patent Document 3), and subsequently the SAFHSTM (registered trademark) ultrasound bone fracture treatment machine was developed by Exogen, Inc. In clinical trials, SAFHSTM has been proven to accelerate healing of tibial tuberosity fractures (Non-Patent Document 3) and distal radius fractures (Non-Patent Document 4). Pittenger et al. have also shown that undifferentiated mesenchymal cells can be differentiated into adipocytes, chondrocytes, and osteocytes in vitro, and the medium composition that leads to differentiation into these three cell types is also known (Non-Patent Document 5, Non-Patent Document 6). Furthermore, it has been reported that bone differentiation is promoted when undifferentiated mesenchymal cells are cultured in bone differentiation inducing medium and then irradiated with ultrasound using the SAFHSTM (SAFHSTM) (Non-Patent Document 7).

Various irradiation conditions have been examined for the effect of ultrasound on cultured cells, and in particular, many studies have shown that ultrasound irradiation devices are effective for cell maturation by applying low-power pulses at the bottom of a culture chamber (Non-Patent Document 8). The inventors have found that when undifferentiated mesenchymal cells are cultured in a medium for inducing chondrogenic differentiation to produce artificial cartilage, ultrasound irradiation during culture markedly promotes the differentiation of undifferentiated mesenchymal cells into chondrocytes (Patent Document 4).

Non-Patent document 17 demonstrates that stem cells from human exfoliated deciduous teeth-conditioned medium (SHED-CM) is a promising treatment for amyotrophic lateral sclerosis based on its neuroprotective effects.

Patent document 5 discloses a composition for regenerative treatment comprising a dental pulp-derived stem cell culture supernatant that does not contain the dental pulp-derived stem cell itself for use in the treatment of Alzheimer's disease.

### [Prior Art Documents]

### [Patent Documents]

Patent document 1: JP 2002-281962
Patent document 2: Japanese Patent No. 6296622
Patent document 3: U.S. Patent No. 4,530,360
Patent document 4: Japanese Patent No. 4676679
Patent document 5: WO 2020/130038 A

### [Non-patent documents]

Non-Patent document 1: Atala A. and Lanza R ( 2021-12-31) Handbook of Stem Cells.Academic Press P 452, ISBN 978-0-12-385943-3
Non-Patent document 2: Parvizi et al.: J Orthop. Res., 17(4):488-94, 1999
Non-Patent document 3: Heckman et al.: J Bone and Jiont Surg, 76A: 25-34, 1994
Non-Patent document 4: Kristiansen et al.: J Bone and Joint Surg, 79A: 961-973, 1997
Non-Patent document 5: Pittenger MF et al.: Multilineage potential of adult human mesenchymal stem cells. SCIENCE 284(2): 143-147, 1999
Non-Patent document 6: Pittenger MF et al. : Mesenchymal stem cell : cell biology to clinical progress. nature partner journal(npj)|regenerative medicine . Published:02 Dec. 2019.Open Access
Non-Patent document 7: Okada et al : The 4th Japanese Society for Tissue Engineering, Kawasaki, July 6-7, 2001.
Non-Patent document 8: S.-G.Yan et al.Med.Hypotheses,76:4-7,2011
Non-Patent document 9: Miura et al. SHED: stem cells from human exfoliated deciduous teeth. Proceedings of the National Academy of Sciences (2003)Vol.100, 5807-5812
Non-Patent document 10: Matsubara,K.,et al : Secreted ectodomain of sialic acid-binding Ig-like lectin and monocyte chemoattractant protein-1 promote recovery after rat spinal cord injury by altering after rat spinal cord injury by altering macrophage polarity.,J.Neuroscience 35(2015):2452-2464.
Non-Patent document 11: Drago,D.,et al : The stem cell secretome and its role in brain repair.,Biochimie 95.12(2013):2271-2285.
Non-Patent document 12: Shimojima,C. et al; Conditioned medium from the stem cells of human exfoliated deciduous teeth ameliorates experimental autoimmune J.Immunol 196(2016):1-8
Non-Patent document 13: Matsubara,K.et al.J.Neurosci.11,2015.35(6): 2452-2464)
Non-Patent document 14: Clinical Neurology 2011:51:1195-1198
Non-Patent document 15: Pollari,E. etal:In Vivo electropysiological measurement of compound muscle action potential from the forelimbs in models of motor neuron Neuroscience,15:2018 doi:10.3791/57741
Non-Patent document 16: Osuchowski,M., et al: Noninvasive model of sciatic nerve conduction in healthy and septic mice; rehabilitation and normative data . Muscle Nerve. 2009 Oct40(4): 610-6. doi;10.1002/mus.21284
Non-Patent document 17: Ueda Tomoyuki ET AL: "Stem Cells From Human Exfoliated Deciduous Teeth-Conditioned Medium (SHED-CM) is a Promising Treatment for Amyotrophic Lateral Sclerosis", Frontiers in pharmacology, vol. 13, 3 February 2022 (2022-02-03)

### Summary of the Invention

### Problems to be Solved by the Invention

The problem to be solved by this invention is to provide a composition for treatment of intractable neurological diseases and a manufacturing method thereof. In particular, it is a problem that this invention seeks to solve to enable the treatment of amyotrophic lateral sclerosis (ALS) and Alzheimer's disease.

### Means for Solving the Problems

The inventors of this application have made a careful study and found that the conditioned medium obtained by culturing human deciduous tooth pulp stem cells (stem cells from exfoliated deciduous teeth; hereinafter referred to as deciduous tooth pulp stem cells or SHED), which are medical waste, in serum-free medium under ultrasound irradiation, can be used to treat amyotrophic lateral sclerosis (ALS) and Alzheimer's disease and thereby made the invention according to the claims.

Dentin pulp stem cells have been identified as a novel stem cell population with self-renewal and multipotency similar to bone marrow stem cells (BMSC). These cells are a neural crest-derived cell population with properties similar to those of the nervous system and are highly responsive to induction of neuronal differentiation. Because they are tissue stem cells, they are safe for transplantation and pose very few ethical problems (Non-Patent Document 9).

The supernatant fluid (conditioned medium) generated when culturing stem cells as described above contains more than several thousand kinds of protein components and is rich in cytokines, chemokines, exosomal proteins, and other signaling substances that are key to cell activity. For example, Epidermal Growth Factor (EGF), Fibroblast Growth Factor (FGF), Platelet-Derived Growth Factor (PDGF), Hepatocyto Growth Factor (HGF), Transforming growth Factor (TGF), vascular endothelial growth Factor (VEGF), and other growth factors, MCP-1 (Monocyte Chemotactic protein-1, a monocyte chemotactic protein), Siglec-9 ( Sialbinding immunoglobulin-type lectins-9, sialic acid-binding Ig-like lectin-9), and exosomes are known to be included (Non-Patent Document 10, 11). And it has been demonstrated from various studies that culture supernatants containing such cytokines, chemokines, exosomal proteins, etc. have functions to repair damaged tissues and to protect tissues (Non-Patent Document 10, 11).

As mentioned above, conditioned medium do not cause immune rejection because they do not contain cells themselves. Therefore, conditioned medium prepared from other people's stem cells can be used without concern for rejection, and the range of application is extremely high. In addition, if the culture supernatant is stored in advance, it can be used immediately when needed, which is highly convenient.

The components of conditioned medium differ depending on the type of stem cells. It has been found that culture supernatants made from deciduous dental pulp stem cells (hereinafter referred to as SHED) (deciduous dental pulp stem cell conditioned medium, hereinafter referred to as SHEDCM) contain a particularly large amount of protein components, especially cytokines, chemokines, and exosomal proteins that are beneficial for nerve regeneration (Non-patent document 11, 12).
SHEDCM is defined as a cell-free culture medium obtained by culturing SHED. The compositions of the present invention contain culture supernatant as an active ingredient, but do not contain cells. The compositions of the present invention are clearly distinguished from various compositions containing stem cells, as well as SHED itself, by the feature that they do not contain cells.

The cytokines included in SHEDCM are MCP-1 (monocyte chemotactic protein), Siglec-9 (sialic acid-binding Ig-like lectin-9), and HGF (hepatocyte growth factor). MCP-1 is a basic protein of 76 amino acids that exhibits specific migratory activity against monocytes, which are immune cells. MCP-1 is also involved in the expression of adhesion molecules on the surface of monocytes, and is thought to be involved in the migration of monocytes to inflammatory sites, adhesion with endothelial cells, and infiltration into the subendothelium. And MCP-1 can promote tissue infiltration of lymphocytes, which are immune cells, in inflammation (accumulate NK cells and NKT cells in inflammation) (Non-Patent Document 12).

Siglec-9 cooperates with MCP-1 to suppress inflammatory macrophage (M1 macrophage) activity and simultaneously convert polarity to anti-inflammatory and regenerative macrophages (M2 macrophages) (Non-Patent Document 13).

HGF is an endogenous tissue regeneration and repair factor that supports the body's natural healing ability. HGF has various effects such as anti-inflammatory, antioxidant, anti-fibrotic, angiogenesis-promoting, and cell death inhibitory actions, and has been reported to be useful for ALS (Non-Patent Document 14).

### Effects of Invention

The composition for treating intractable neurological diseases of the present invention has a therapeutic effect on intractable neurological diseases, especially amyotrophic lateral sclerosis (ALS) and Alzheimer's disease.

### BRIEF DESCRIPTION OF THE DRAWINGS

Fig.1: A diagram showing the method of ultrasound irradiation during SHED culture.
Fig.2: A table showing the effects of ultrasound irradiation throughout the culture period on the concentrations of the three cytokines in the culture supernatant at the end of the culture period.
Fig.3: A graph showing the relationship between the output of ultrasound irradiated during culture and the concentration of the cytokine HGF in the conditioned medium at the end of the culture period.
Fig.4: The figure shows the protocol of an experiment to examine the effect of SHEDCM on a mouse model of amyotrophic lateral sclerosis.
Fig.5: The graph shows the changes in the compound muscle action potentials of each group of mice when SHEDCM or saline solution was administered to the amyotrophic lateral sclerosis model mice and control mice.
Fig.6: The graph shows the survival rate of each group of mice when SHEDCM or saline solution was administered to amyotrophic lateral sclerosis model mice and control mice.
Fig.7: The figure shows the method of ultrasound irradiation during incubation in culture flasks.
Fig. 8: This figure shows the method of measuring the range of motion.
Fig. 9: A table showing the results of improvement in the range of motion of patients with amyotrophic lateral sclerosis by SHEDCM treatment.
Fig. 10: A graph showing the HDS-R before and 8 weeks after SHEDCM treatment in patients with Alzheimer's disease.
Fig. 11: A graph showing the time of onset of therapeutic effect when SHEDCM was administered to patients with Alzheimer's disease.
Fig. 12: A graph showing the duration of the therapeutic effect when SHEDCM is administered to patients with Alzheimer's disease.
Fig. 13: A conceptual diagram of the floating culture method, which is a mass production method of H-SHEDCM.
Fig. 14: A diagram showing the effect of H-SHEDCM, R-SHEDCM, and a mixture of the three cytokines on alleviating disease severity in ALS mice.

### MODE FOR CARRYING OUT THE INVENTION

The following is an embodiment of the invention. It goes without saying that various changes and modifications can be made to the extent that they do not depart from the technical scope of the invention.

### EXAMPLE 1

### Production of milk stem cells from human exfoliated deciduous teeth (SHED)

### (1) Collection of SHEDs

SHEDs are sorted as adherent cells from dental pulp tissue collected from deciduous teeth of humans that have dropped out. After disinfecting the tooth of a naturally deciduous tooth with chlorohexidine solution, the crown of the tooth is divided and the pulp tissue is collected with a dental reamer. Povidone-iodine solution (Isodine (registered trademark) solution) may be used instead of chlorohexidine solution.

### (2) Enzyme treatment

( The pulp tissue collected in (1) above is suspended in basic medium (Dulbecco's Modified Eagle's Medium containing 10% bovine serum and antibiotics (sometimes referred to as "DMEM")). The cells are suspended in Dulbecco's Modified Eagle's Medium (Dulbecco's Modified Eagle's Medium, sometimes referred to as "DMEM") and treated with 2 mg/ml of collagenase and disposed at 37°C for 1 hour. Centrifuge (600-5000 x g for 5 min) and collect the pulp tissue and pulp cells after enzyme treatment.

### (3) Cell culture

The pulp tissue and pulp cells collected as described above are suspended in 4 cc of Dulbecco's Modified Eagle's Medium (DMEM) or mesenchymal stem cell medium containing 5 to 15% bovine serum by volume and 50 to 150 units/ml of antibiotics, and seeded into adherent cell culture dishes, 6 wells. The cells are then incubated in a 5-volume% carbon dioxide (hereinafter referred to as _{CO2} ) atmosphere at approximately 37°C. When the cells reach sub-confluent (i.e., cells occupy approximately 70% of the surface area of the culture vessel) or confluent, cells are treated with 0.05 volume% trypsin-EDTA at 37°C for 5 minutes. Dental pulp-derived stem cells detached from the dish are seeded into a 10 cm diameter adherent cell culture dish for expansion culture.

Passage cultures may be repeated, and cell cultures should be grown to the required cell number (e.g., approximately 1 × 10⁷ cells/ml) by conducting 1 to 15 passages. After the above culture, the cells may be collected and stored.

### (4) Collection of cells

After detaching the cells from the culture vessel by trypsin treatment, the cells (adherent cells) are collected by centrifugation to recover SHED. The centrifugation conditions should be 600 to 5000 x g, more preferably 750 to 5000 x g.

### Production of SHED derived conditioned medium (SHEDCM):

Next, an example of the production of SHEDCM is described. First, milk pulp-derived stem cells (SHED) obtained by the above method are placed in a culture vessel, and basic medium (medium to which animal serum such as 10% by volume FBS is added as serum, (such as DMEM described above)) is added. The culture is incubated at 37°C for 24 to 48 hours under an atmosphere of 5 volume% CO₂. The medium is then replaced with serum-free DMEM, placed on an ultrasound generator (DISCIVER, SONIC SOAC), and incubated for another 24 to 72 hours while being irradiated with ultrasound waves, and then the culture supernatant is collected. The ultrasound waves should have a frequency of 20 KHz to 10 MHz, a burst width of 10 µsec to 1 msec, a repetition rate of 5 Hz to 10 KHz, and an output of 5 to 120 mW/cm². To completely remove the pulp-derived stem cells from the collected culture supernatant, the collected culture supernatant is centrifuged at 600 to 5000 × g for 3 to 7 minutes to obtain a treated SHEDCM that contains no pulp-derived stem cells (SHED is removed).

Another method of removing SHED is to pass the cells through a membrane that does not allow passage of SHED to obtain a treated SHEDCM that is free of SHED (i.e., free of pulp-derived stem cells).

Although there is no limit to the number of generations of SHED used to produce SHEDCM, it is preferable to use 5 to 15 in terms of the ability to improve or prevent the target tissue and the range of target tissue types.

### EXAMPLE 2

The SHEDs obtained by the method of Example 1 were divided into two groups, and their ability to induce stem cell differentiation by ultrasound irradiation was verified using specific cytokines as indicators. The first group (Group 1) was the non-irradiated group, and the second group (Group 2) was the ultrasound-irradiated group. The number of repetitions for each group was 5 times (n=5), and culture was performed in basic medium (DMEM; medium with 10 volume % FBS or other animal serum added as serum) at 37°C under 5% CO2, and the basic medium was changed every 3 days.

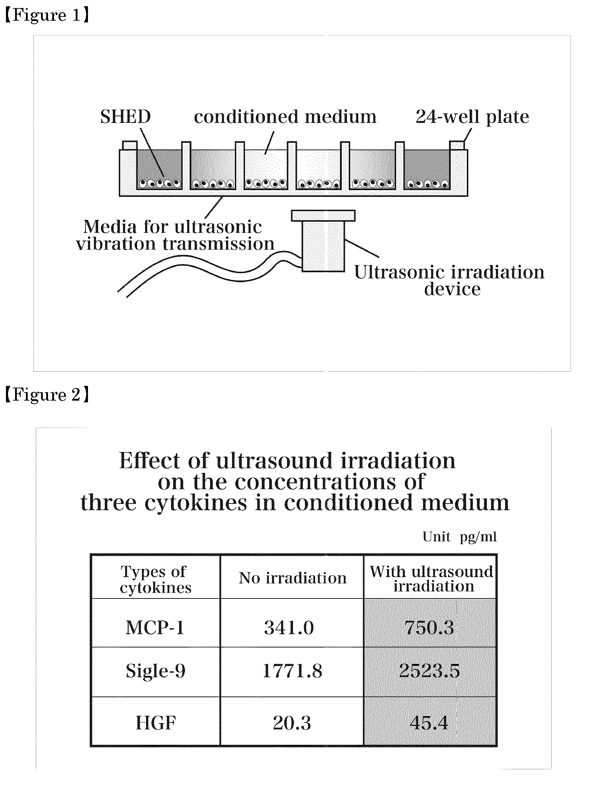

SHEDs cultured in this manner were placed in 24-well plates and grown in the above basic medium for 2 weeks. After that, the culture supernatant of each well was removed and replaced with serum-free DMEM. The 24-well plate containing SHED of Group 2 was placed on an ultrasound wave generator (DISCIVER, SONIC SOAC) and irradiated with ultrasound for 48 hours in the way in which the ultrasound frequency was 1.5 MHz, the burst width was 200 micro second, the repetition cycle was 1.0 KHz and the ultrasound output was 120mW/cm2 (Figure 1). Group 1 SHEDs were cultured in the same manner as Group 2 SHEDs, except that Group 1 SHEDs were not irradiated with ultrasound.

The conditioned medium of Group 1 and Group 2 were then collected, centrifuged at 300 × g for 5 min, and the conditioned medium was filtered through a 0.22 mm syringe filter to obtain SHED conditioned medium SHEDCM. The concentrations of MCP-1, Siglec-9 and HGF in the SHED culture supernatant were measured by microarray method The results are shown in Figure 2. The results are shown in Figure 2. The culture supernatant obtained by sonication contained higher concentrations of MCP-1, HGF, and Siglec-9 than the conditioned medium obtained by non-irradiation.

### EXAMPLE 3

The SHEDs obtained by the method of Example 1 were divided into four groups, and the relationship between the ultrasound output of irradiation and the concentration of HGF accumulated in the culture supernatant was verified. The number of repetitions for each group was 5 (n=5), and the culture was performed in the same manner as in Example 2. The method of ultrasound irradiation was the same as in Example 2, but the output of ultrasound irradiated was changed to 10 mW/cm² for the first group to 50 mW/cm² for the second group, to 100 mW/cm² for the third group,^{,} and to 120 mW/cm² for the fourth group.

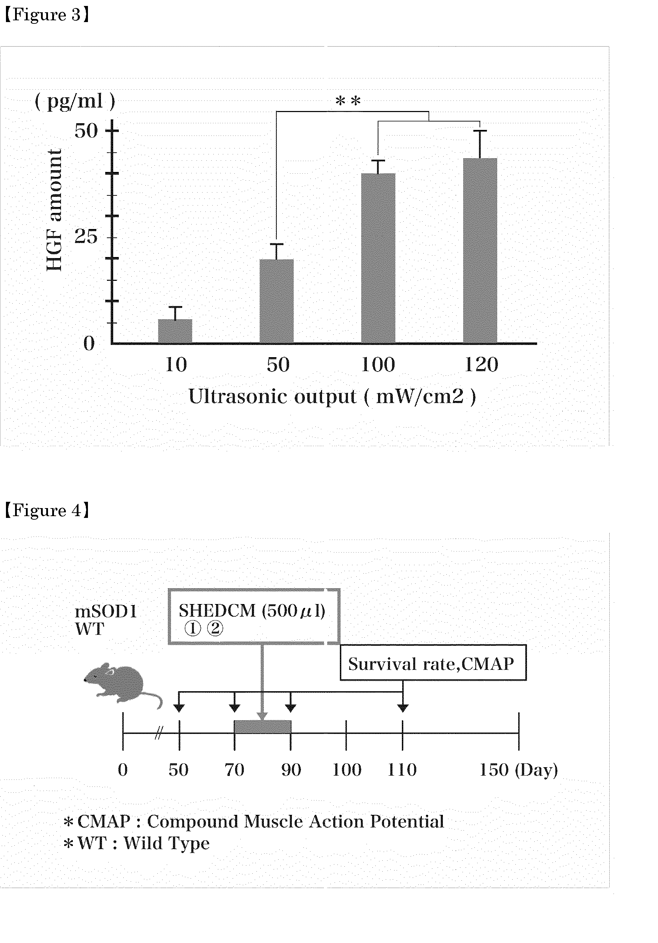

The conditioned medium was collected as in Example 2, and the concentration of HGF in the conditioned medium obtained was measured by the microarray method. The results are shown in Figure 3. When ultrasound irradiation was performed at an output of 10 to 120 mW/cm², a significant difference was observed between the HGF concentration at 50 mW/cm² and that at 100 mW/cm² and 120 mW/cm² (1% level, by T test), with the highest HGF production at an output of 120 mW/cm².

### EXAMPLE 4

The effect of SHEDCM on amyotrophic lateral sclerosis model mice was verified by animal experiments. The ALS model mice (mSOD1) used in the experiment were recombinant mice with the G93A mutant SOD1 gene, which develop the disease at around 70 days of age (P70) and die by P150. In this experiment, P50 to P150 were used for observation. The protocol of the experiment is shown in Figure 4.

The following four groups were established: R-SHEDCM refers to culture supernatants prepared without sonication, H-SHEDCM refers to culture supernatants prepared with sonication, and n refers to the number of experimental replicates.
Control group 1: WT (healthy mice) n=5
Control group 2: mSOD1 (ALS mice) n=5
Experimental group 1: Non-irradiated group (mSOD1 administered with R-SHEDCM) n=5
Experimental group 2: Ultrasound irradiated group (mSOD1 administered with H-SHEDCM) n=5
R-SHEDCM and H-SHEDCM were prepared as described in Example 2.

In the experimental group, ALS mice (mSOD1) were administered 0.5 ml of R-SHEDCM or H-SHEDCM solution via tail vein per day for 20 consecutive days from P70 (before onset) to P90 (after onset). The control group (WT and mSOD1) received the same volume of saline solution.

The survival condition was observed until P150, and at P50, P70, P90, and P110, CMAP (Compound Muscle Action Potential) of the lower limbs was measured by needle myopotential testing using the method described in Non-Patent Document 15.

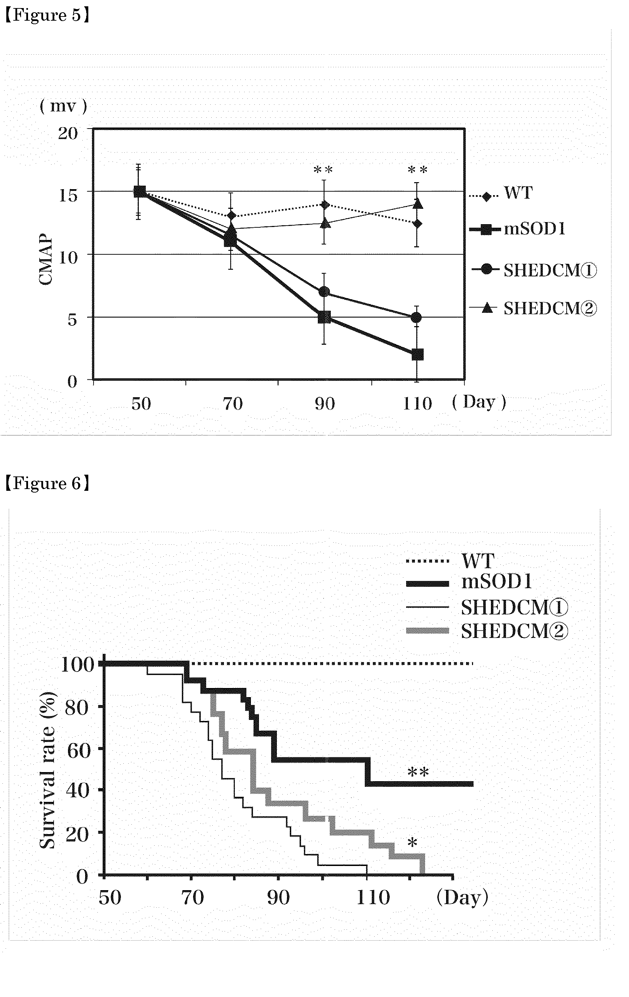

The results of the experiment were as follows. The results of the measurement of compound muscle action potential (CMAP) are shown in Figure 5. In control group 2 (ALS mice administered with saline) and experimental group 1 (ALS mice administered with R-SHEDCM; SHEDCM ① in Figure 5), the compound muscle action potential continuously decreased, reaching less than 5 mv on day 110. There was no significant difference between the two groups. On the other hand, the compound muscle action potentials of control group 1 (normal mice administered with saline) and experimental group 2 (ALS mice administered with H-SHEDCM; SHEDCM ② in Figure5) remained above 10 mv until day 110, and there was no significant difference between the two groups. And there was a significant difference (1 percent level, by T-test) in compound muscle action potentials between the groups consisting of the control group 2 and the experimental group 1 and other groups consisting of the control group 1 and the experimental group 2.

The normal range of compound action potentials in mice is roughly in the range of 10 mV to 20 mV (Non-Patent Document 16). Composite muscle action potentials in all four experimental groups remained in the normal range until day 70, but then declined in the mSOD1 and non-irradiated groups, falling below the normal range by day 90 and below 5 mV by day 110. On the other hand, WT and the ultrasound-irradiated group maintained the normal range until day 110.

The supernatant produced without ultrasound irradiation (R-SHEDCM) failed to suppress the decrease in compound action myoelectric potentials in ALS mice. The ultrasound-irradiated culture supernatant (H-SHEDCM) suppressed the decrease in compound muscle action potentials (CMAP) in ALS mice and maintained CMAP at the same level as normal mice.

The survival rate of mice in each group is shown in Figure 6. Almost 100% of the mice in all four groups survived at P70, while the survival rate at P110 was 100% in WT, 0% in control group 2, 25% in experimental group 1 (SHEDCM ① in Figure 6), and 50% in experimental group 2 (SHEDCM ② in Figure 6). The survival of ALS mice was prolonged by the conditioned medium prepared without ultrasound irradiation (R-SHEDCM). However, the conditioned medium prepared with ultrasound irradiation (H-SHEDCM) significantly outperformed R-SHEDCM, with a significant difference at the 1% level. The significance of the difference between the H-SHEDCM and the R-SHEDCM was statistically significant at the 1% level. The significance test method was the T-test.

### EXAMPLE 5

### Preparation of H-SHEDCM using flasks

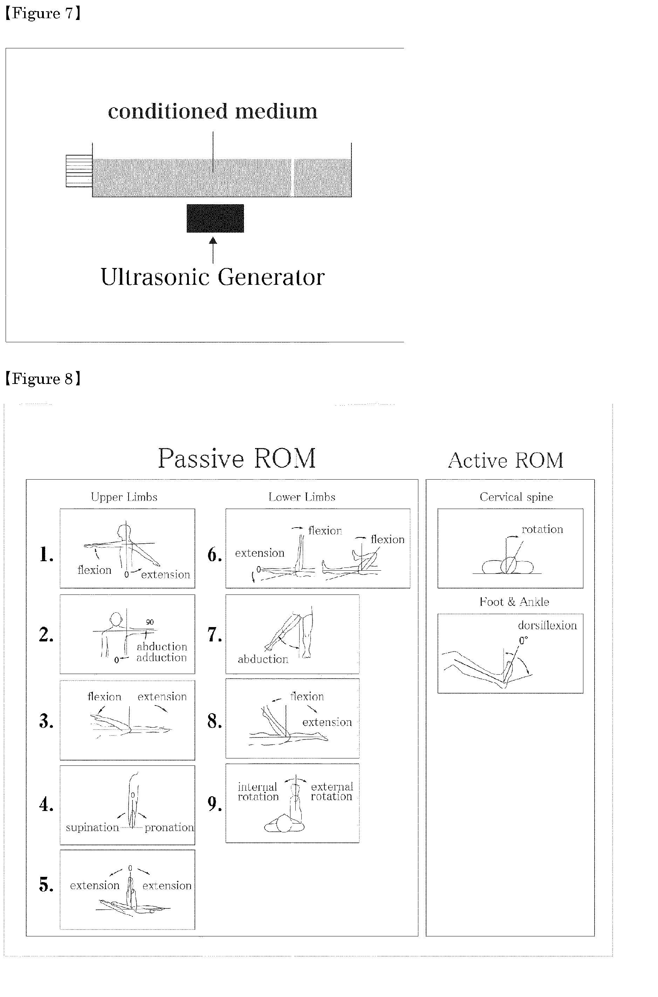

In a culture flask (Nunc (registered trademark) EasYFlask (registered trademark) Cell Culture Flask 159934, 70 mL capacity, 225 Cm² culture area), 30 mL of the basic medium (DMEM) described in Example 2 was placed and SHED prepared by the method of Example 1 was seeded at 1.0 × 10⁴ cells/mL.. The culture was incubated at 37°C and 5% CO₂ for 2 weeks with timely replacement of the culture medium. The culture flasks were then placed on an ultrasound generator (DISCIVER, SONIC SOAC) and irradiated with ultrasound pulses at a frequency of 1.5 MHz, burst width of 200 psec, repetition rate of 1.0 KHz, and ultrasound output of 120 mW/cm2 for 48 hours (Figure 7). The culture medium was then collected from the flask and aliquoted into 50-mL centrifuge tubes, and the precipitates were removed by centrifugation (2000 rpm, 3 min) to obtain the conditioned medium (H-SHEDCM). R-SHEDCM was prepared by the same method as above, except that it was not sonicated.

### EXAMPLE 6

The therapeutic effect of SHEDCM on amyotrophic lateral sclerosis was examined. H-SHEDCM prepared by ultrasound irradiation was used for a short period of time for ALS patients for whom no treatment currently exists, and not only stopped the progression of the disease but also successfully improved the symptoms. The results of the SHEDCM treatment are summarized below. R-SHEDCM and H-SHEDCM were prepared as described in Example 5.

Patient (68 years old, male) was diagnosed with ALS in June 2020. The patient was then transferred to a hospital specializing in ALS, where he was monitored, but the progression of his symptoms did not stop. The patient then requested treatment with SHEDCM, and was referred to the inventor in January 2021.

The patient showed characteristic symptoms of ALS. In particular, the decline in respiratory function (% lung capacity, 66.5% (June 2020) to 46.1% (August 2020)) was severe, and the patient also had significant limb spasticity, confirming the rapid deterioration of the disease.

From January 2021 to August of the same year, R-SHEDCM was administered intravenously (240 ml/week). During this period, there was no change in his symptoms, and his respiratory function decreased (room air, Spo2<90%) and his limb spasticity progressed. Therefore, in September of the same year, H-SHEDCM infusion (240 ml/week) was started. 1 week after the start of H-SHEDCM infusion treatment, spasticity was relieved and automatic joint range of motion was rapidly increased in the extremities and fingers (especially thumb and little finger), and Automatic movement and respiratory function continued to improve.

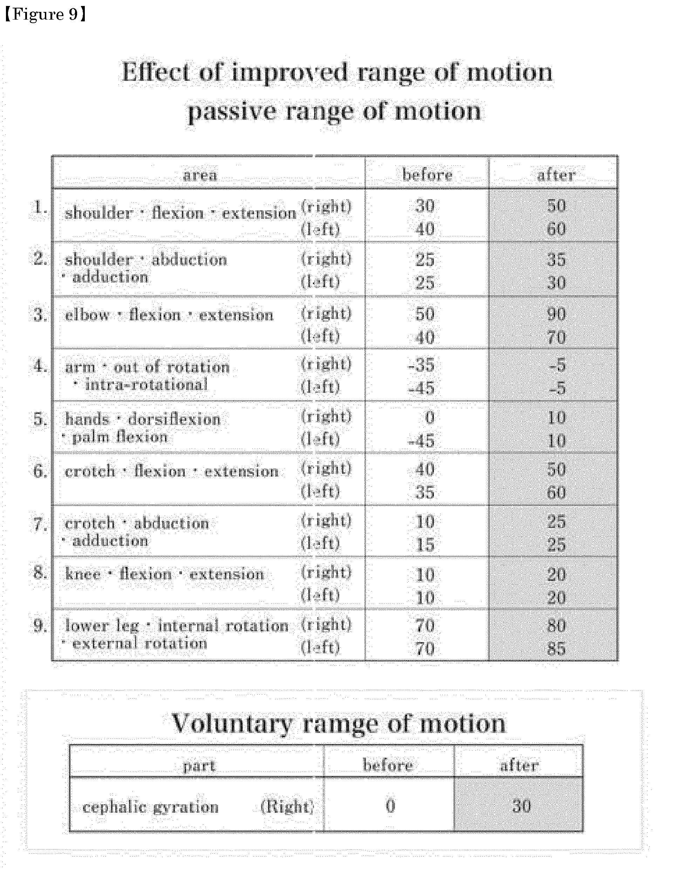

The patient's range of motor movement was measured before the start of treatment (January 2021) and after 3 months of intravenous administration of H-SHEDCM (December 2021). Figure 8 shows how the range of motion was measured. Figure 9 shows the patient's range of motion before treatment and after 3 months of H-SHEDCM administration. The passive range of motion is defined as the range of motion that can be moved by another person's hand, and the voluntary range of motion is defined as the range of motion that can be moved by the patient's will (voluntary motion). Range of motion is measured by evaluating the angle of rotation of an extremity or finger around a joint axis. A joint angle meter (Angle Meter (Mr. Kaminaka) Matsuyoshi Medical Instruments) was used for the measurement.

The numbers in Figure 9 indicate the angles. The numbers in column "Before" indicate the joint range of motion measured by the above method before treatment, and the numbers in column "After" indicate the joint range of motion after 3 months of H-SHEDCM administration.

In the case of "shoulder flexion/extension," the numbers are positive in the anterior direction and negative in the posterior direction, with the perpendicular line position at 0 degrees. The patient's right arm was flexed at 30 degrees in the forward direction before treatment, but after 3 months of H-SHEDCM administration, the arm was able to move to a position of 50 degrees if the patient put his hand on it.

The position of the arm's external rotation and internal rotation is defined as 0 degrees when the upper arm is vertical (parallel to the body), negative numbers when the arm is rotating inward, and positive numbers when the arm is bending outward. Before treatment, the patient's right arm was inverted from a vertical position (0 degrees) to a position of 35 degrees of inward rotation (-35 degrees); after 3 months of H-SHEDCM administration, the patient could move it 30 degrees outward (-5 degrees) with the help of his hand.

In all the measured parameters, the patient's range of motion was greater after 3 months of treatment with H-SHEDCM than before treatment.

In January 2022, the patient's respiratory function was improved with room air and SpO2 >97%.

As of March 2022, the patient continues to receive H-SHEDCM intravenously, and his range of motion has further improved.

The benefits of H-SHEDCM are clear. Progressive atrophy of respiratory muscles and spasticity of limbs are characteristic symptoms of ALS caused by inflammation and degeneration of motor neurons. Although the progression of the disease can be slowed after onset, there have been no previous cases of successful arrest or recovery of the progression. The fact that this ALS treatment improved respiratory function, which was deteriorating rapidly, and continued to improve limb motor range of motion indicates that H-SHEDCM has high anti-inflammatory and neuroregenerative effects.

### EXAMPLE 7

The therapeutic effect of H-SHEDCM on Alzheimer's disease was tested. A total of 20 patients with Alzheimer's disease, 16 females and 4 males, were included in the study. The mean age of the patients was 85.5 years. Ten of these patients were randomly selected and treated with H-SHEDCM created by ultrasound irradiation, and the other 10 patients were treated with R-SHEDCM created without ultrasound irradiation. Administration was by nasal drip of 0.5 ml of H-SHEDCM or R-SHEDCM 3x concentrated solution per dose. Dosing was done twice per day. This was continued for 8 weeks. R-SHEDCM and H-SHEDCM were prepared as described in Example 5.

Patients' cognitive function was assessed by the revised Hasegawa Dementia Scale (HDS-R). The cognitive function data of these patients were divided into the following three groups.

Group 1 is the control group, which consists of the cognitive function data of all patients immediately before the start of treatment. 20 patients are included, so the number of repetitions (n) is 20.

Group 2 is the group that received the SHEDCM without ultrasound irradiation (R-SHEDCM). The number of repetitions is 10.

Group 3 is the group that received the SHEDCM created by ultrasound irradiation (H-SHEDCM). The number of repetitions is 10.

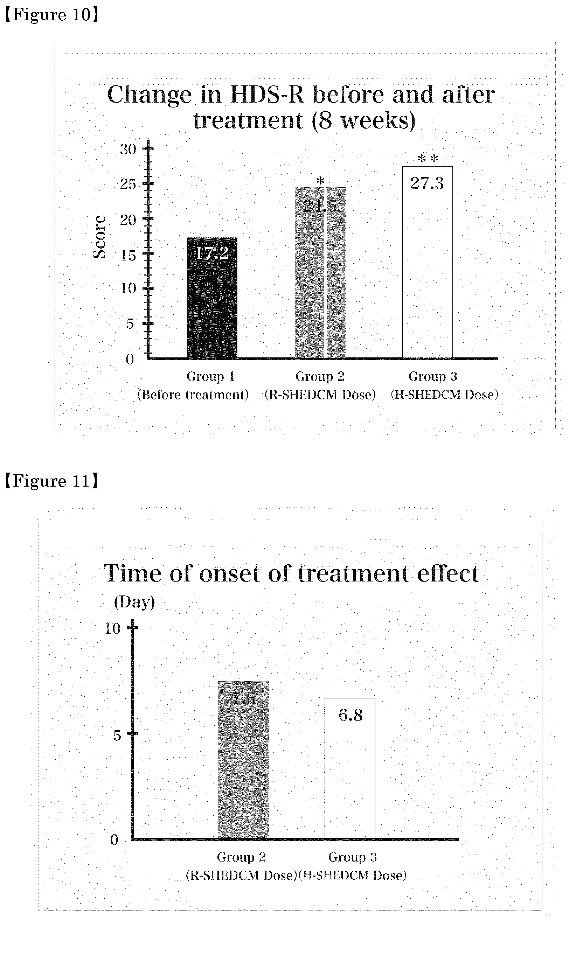

The HDS-R scores before treatment and after 8 weeks for groups 2 and 3 are shown in Figure 10: 17.2 for group 1, 24.5 for group 2, and 27.3 for group 3. At the end of treatment (8 weeks after the start of treatment), both groups 2 and 3 showed significant differences in effectiveness for patients with Alzheimer's disease (AD) at the 5% and 1% level (t-test), respectively, compared to the pre-treatment group. Group 3 showed a greater treatment effect than group 2.

The time of onset of efficacy, or the time (in days) when improvement in HDS-R was confirmed, averaged 7.5 days in group 2 and 6.8 days in group 3, with no significant difference between the two groups (Fig. 11). After the onset of the effect, no decrease in HDS-R was observed during the period of intranasal administration of SHEDCM.

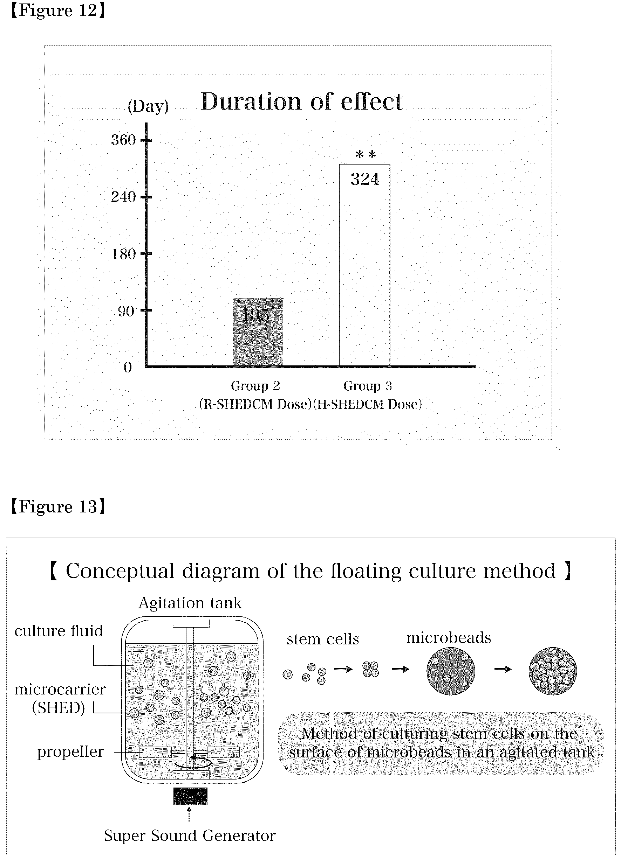

The duration of effect, i.e., the period of time (in days) that the effect lasted after the end of treatment, averaged 3.5 months in group 2 and 10.8 months in group 3, about three times longer than in group 2 (Fig. 12).

The culture supernatant prepared with ultrasound irradiation (H-SHEDCM) was more effective in treating Alzheimer's disease than the culture supernatant prepared without ultrasound irradiation (R-SHEDCM), and the duration of the effect was longer. Cognitive decline due to Alzheimer's disease is said to be caused by neurodegeneration in the hippocampus due to deposition of β-amyloid. In detail, the neurodegeneration results in a decrease in reactive acetylcholine production (reduced neurological function), which is responsible for the decline in memory capacity and a decrease in the number of nerve cells.

The treatment effect was higher in group 3 (H-SHEDCM group) than in group 2 (R-SHEDCM group), but there was no difference in the time of effect onset, suggesting that SHEDCM may have improved the ability to produce reactive acetylcholine. On the other hand, the large difference in duration of effect between groups 2 and 3 suggests that the ultrasound-irradiated culture supernatant may have increased the number of neurons.

Currently, the most promising treatment for Alzheimer's disease, adducumumab (unapproved), must be administered intravenously. However, the culture supernatant therapy can be administered intranasally at home. The ability to administer the treatment at home is a notable advantage because it greatly reduces the burden on patients and their caregivers.

### EXAMPLE 8

Mass production of H-SHEDCM by floating culture using microcarrier beads SHED prepared by the method of Example 1 was seeded in the basic medium (DMEM) described in Practical Example 2 at 1.0 x 10⁴ cells/mL, and microcarrier beads (GE Healthcare, Cytodex (registered trademark) 3) were added at 1.0 x 10³ beads/mL to the animal cell culture device (Animal Cell Culture Equipment (Co. Biot, BCP-15NP4) , and the SHED was cultured for 2 weeks at 37°C, 5% CO₂, and agitation speed of 15 rpm, the culture medium being replaced with fresh medium in a timely manner(Fig. 13). After 2 weeks of incubation, the culture medium was replaced with serum-free DMEM and the SHED was grown for 48 hours being irradiated with ultrasound, frequency of 1.5 MHz, burst width of 200 psec, repetition rate of 1.0 KHz and ultrasound output 120 mW/cm2, using an ultrasound apparatus (SHARP (registered trademark), ultrasound oscillator UT-304N/604N/1204). The ultrasound generator was placed at the bottom or side of the culture tank. After that, the culture tank was removed from the device, and the culture medium was dispensed into a 50-mL centrifuge tube in a clean bench, centrifuged at 2000 rpm for 3 minutes, and microbeads, cells, and sediment were removed to obtain the culture supernatant (H-SHEDCM). After 2 weeks of culture, SHED attached to the microcarrier beads was detached using trypsin, and the cell count of SHED collected in suspension in the culture medium was measured with a cell counter, and the cell count was 1.08 x 108 cells/mL.

### EXAMPLE 9

### Comparison of H-SHEDCM and recombinant cytokines using ALS model mice

The effects of H-SHEDCM and a mixture of three major cytokines, Siglec-9, MCP-1 and HGF, on ALS severity in ALS model mice were compared.

The ALS model mice used were the same as the model mice used in Example 4; H-SHEDCM and R-SHEDCM were prepared as described in Example 2. The three cytokines used, Siglec-9, MCP-1, and HGF, were produced by genetic recombination and purchased from Cosmo Bio Co.

Nine 90-day-old ALS mice (mSOD1) were used, three of which received 500 µL of H-SHEDCM, another three received 500 µL of R-SHEDCM, and another three received 100 µg of Siglec-9, 500 µg of MCP-1 and 20 µg HGF mixture was administered by injection into the tail vein. Symptoms of each mouse were determined by symptom severity score on day 7 after administration. The three mice treated with H-SHEDCM are referred to as group 1, the three mice treated with R-SHEDCM as group 2, and the three mice treated with a mixture of the three cytokines as group 3.

The symptom severity scores are as follows
0: No disease
1: Loss of tail tone
2: Hind limb weakness
3: Paralysis of hind limbs
4: Paralysis of hind limbs and forelimbs
5: Dying or dead

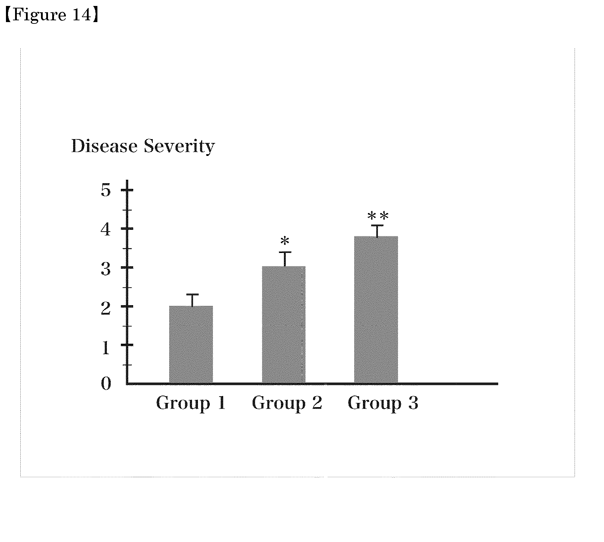

The mean symptom severity for each group on day 7 of treatment was 2.0 for group 1, 3.0 for group 2, and 3.3 for group 3. T-tests showed a significant difference between groups 1 and 2 at the 5% level and between groups 1 and 3 at the 1% level (Figure 14).

As shown in Example 2, Siglec-9, MCP-1, and HGF are major components in SHEDCM, and their concentrations in the culture supernatant were increased by sonication in SHED culture. However, as shown in this comparison, the effect of a mixture of these three cytokines in alleviating disease severity in ALS mice did not extend to H-SHEDCM. Amounts of Siglec-9, MCP-1, and HGF in 500 µL of H-SHEDCM administered to the group 1 were approximately 600 pg, 370 pg, and 23 pg, respectively. On the other hand, the amounts of Siglec-9, MCP-1, and HGF administered to the group 3 are 100 µg, 500 µg, and 20 µg, respectively, which are much larger than the amounts of Siglec-9, MCP-1, and HGF in 500 µL of H-SHEDCM administered to the group 1. Nevertheless, the disease severity of the group3 was much more severe than that of the group 1. This indicates that these three major cytokines alone are not sufficient to alleviate disease severity in ALS mice.

The conditioned medium contains 1,000 to 2,000 cytokines. Although it is virtually impossible to determine the effect of ultrasound stimulation on the concentrations of these cytokines in the conditioned medium, it is thought that the presence of trace amounts of cytokines other than the major components in the conditioned medium is important for the expression of the effect. The ultrasound irradiation by the method of the present invention during culture is thought to foster a composition in the culture supernatant that exerts an effect on alleviating the severity of the disease in ALS mice.

### INDUSTRIAL APPLICABILITY.

The compositions for the treatment of intractable neurological diseases of the present invention will contribute to the creation of the world's first therapeutic agent for ALS, for which there is still no effective treatment. Furthermore, it will also contribute to the creation of a highly effective therapeutic agent for Alzheimer's disease. It is also expected to be effective in the treatment of intractable neurological diseases other than these two diseases.

## Claims

1. A composition suitable for the treatment of amyotrophic lateral sclerosis (ALS) and Alzheimer's disease, comprising culture supernatant obtained by culturing deciduous dental pulp stem cells in serum-free medium under ultrasound irradiation, in which the ultrasound irradiation is **characterized in that** the ultrasound frequency is 1.5 MHz, the burst width is 200 psec, the repetition rate is 10 KHz, the ultrasound output is 50 to 120 mW/cm2.

2. A method for manufacturing a composition suitable for the treatment of amyotrophic lateral sclerosis (ALS) and Alzheimer's disease, wherein the composition contains culture supernatant obtained by culturing deciduous dental pulp stem cells under ultrasound irradiation using serum-free medium, and wherein the ultrasound frequency is 1.5 MHz, burst width is 200 psec, the repetition cycle is 10 KHz, and the ultrasound output is 50 to 120 mW/cm2.

## Patentansprüche

1. Zusammensetzung, die für die Behandlung von amyotropher Lateralsklerose (ALS) und Alzheimer-Krankheit geeignet ist, umfassend einen Kulturüberstand, der durch Kultivieren von Zahnmark-Stammzellen des Milchgebisses in serumfreiem Medium unter Ultraschallbestrahlung erhalten wird, wobei die Ultraschallbestrahlung **dadurch gekennzeichnet ist, dass** die Ultraschallfrequenz 1,5 MHz beträgt, die Pulsdauer 200 µsec beträgt, die Wiederholungsrate 10 KHz beträgt, die Ultraschallleistung 50 bis 120 mW/cm2 beträgt.

2. Verfahren zur Zubereitung einer Zusammensetzung, die für die Behandlung von amyotropher Lateralsklerose (ALS) und Alzheimer-Krankheit geeignet ist, wobei die Zusammensetzung einen Kulturüberstand enthält, der durch Kultivieren von Zahnmark-Stammzellen des Milchgebisses unter Ultraschallbestrahlung unter Verwendung eines serumfreien Mediums erhalten wird, und wobei die Ultraschallfrequenz 1,5 MHz beträgt, Pulsdauer 200 µsec beträgt, der Wiederholungstakt 10 KHz beträgt und die Ultraschallleistung 50 bis 120 mW/cm2 beträgt.

## Revendications

1. Composition appropriée pour le traitement de la sclérose latérale amyotrophique (SLA) et de la maladie d'Alzheimer, comprenant un surnageant de culture obtenu par culture de cellules souches déciduales de pulpe dentaire dans un milieu exempt de sérum sous irradiation par ultrasons, ladite irradiation par ultrasons étant **caractérisée en ce que** la fréquence des ultrasons est de 1,5 MHz, la largeur de rafale est de 200 µsec, le taux de répétition est de 10 kHz, la sortie des ultrasons est de 50 à 120 mW/cm2.

2. Procédé de fabrication d'une composition appropriée pour le traitement de la sclérose latérale amyotrophique (SLA) et de la maladie d'Alzheimer, ladite composition contenant un surnageant de culture obtenu par la culture de cellules souches déciduales de pulpe dentaire sous irradiation par ultrasons en utilisant un milieu exempt de sérum, et ladite fréquence des ultrasons étant de 1,5 MHz, ladite largeur de rafale étant de 200 µsec, ledit cycle de répétition étant de 10 kHz, et ladite sortie des ultrasons étant de 50 à 120 mW/cm2.
